(19) Europäisches Patentamt
European Patent Office
Office européen des brevets

(11) Veröffentlichungsnummer: **0 193 884**
**B1**

(12) # EUROPÄISCHE PATENTSCHRIFT

(45) Veröffentlichungstag der Patentschrift: **02.08.89**

(21) Anmeldenummer: **86102601.1**

(22) Anmeldetag: **28.02.86**

(51) Int. Cl.⁴: **C07C 43/15**, C07D 307/48, C07C 103/58, C07C 41/28, A61K 7/00, A61K 9/00, C07C 13/28, C07C 5/10, A61K 7/48, A61K 47/00

(54) **Dicyclohexylalkane, ihre Herstellung, diese enthaltende kosmetische und pharmazeutische Zubereitungen und ihrer Verwendung als Ölkomponente.**

(30) Priorität: **01.03.85 DE 3507175**

(43) Veröffentlichungstag der Anmeldung: **10.09.86 Patentblatt 86/37**

(45) Bekanntmachung des Hinweises auf die Patenterteilung: **02.08.89 Patentblatt 89/31**

(84) Benannte Vertragsstaaten: **DE FR GB IT**

(56) Entgegenhaltungen:
**EP-A- 0 135 871**

**ANGEWANDTE CHEMIE, Band 89, Nr. 12, 1977, Seiten 913-914, Verlag Chemie, Weinheim-Bergstrasse, C. RÜCHARDT et al.: "Nachweis einer linearen Beziehung zwischen thermischer Stabilität und Spannungsenergie von Alkanen"**

(73) Patentinhaber: **BASF Aktiengesellschaft, Carl-Bosch-Strasse 38, D-6700 Ludwigshafen(DE)**

(72) Erfinder: **Segnitz, Adolph, Dr., Weimarer Strasse 13, D-4530 Ibbenbueren(DE)**
Erfinder: **Oppenlaender Knut, Dr., Otto-Dill-Strasse 23, D-6700 Ludwigshafen(DE)**
Erfinder: **Naegele, Paul, Wiesenstrasse 9, D-6708 Neuhofen(DE)**

**EP 0 193 884 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Dicyclohexylalkane, ihre Herstellung, diese enthaltende kosmetische und pharmazeutische Zubereitungen und ihre Verwendung als Ölkomponente für kosmetische und pharmazeutische Zwecke.

Bei der Herstellung insbesondere von kosmetischen oder auch pharmazeutischen Zubereitungen für die verschiedensten speziellen Zwecke besteht ein großer Bedarf an medizinischen Weißölen oder flüssigen Paraffinen. Es ist auch schon lange nach einem Ersatz von flüssigem Paraffin gesucht worden. Bei äußerlicher Anwendung auf der Haut ist bei Paraffinöl eine gewisse okklusive Wirkung nachteilig. Deswegen werden in vielen Fällen flüssige Paraffine in äußerlichen Zubereitungen möglichst vermieden. So geht beispielsweise aus der EP-Patentanmeldung 72 988 die Verwendung von 1,3-Dialkyl-cyclohexanverbindungen als kosmetisches Öl hervor.

Aufgabe der vorliegenden Erfindung ist es, neue Öle an Stelle von flüssigen Paraffinen für kosmetische und pharmazeutische Zwecke aufzuzeigen.

Es wurde nun gefunden, daß Dicyclohexylalkane der Formel I

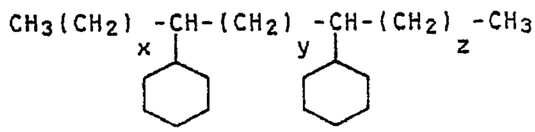

in der x, y und z eine Zahl von 0 bis 24, bevorzugt 0 bis 14, bedeuten, wobei die Summe x + y + z 4 bis 24, bevorzugt 4 bis 14, beträgt, in hervorragender Weise als Ölkomponente in kosmetischen und pharmazeutischen Zubereitungen geeignet sind.

Entsprechend den für x, y und z angegebenen Bedeutungen handelt es sich bei den Verbindungen der Formel I um lineare Alkylketten mit insgesamt 8 bis 28, bevorzugt 8 bis 18, C-Atomen. Die Clycohexylreste befinden sich von der Herstellung her in statistischer Verteilung in der Kette an den sekundären C-Atomen, bevorzugt an den den endständigen Methylgruppen benachbarten C-Atomen. Geminale Cyclohexylgruppen sind von der Herstellung her in der Regel ausgeschlossen.

Die Dicyclohexylalkane der Formel I werden durch Hydrierung der zugrundeliegenden Diphenylalkane - Verbindungen der Formel I mit Benzolresten -mit Wasserstoff unter einem Druck von 90 bis 220 bar und bei Temperaturen von 150 bis 350°C in Gegenwart eines Hydrierkatalysators hergestellt.

Ausgangsverbindungen sind in der Regel keine einheitlichen Verbindungen sondern technische Gemische von Diphenylalkanen mit Kettenlängen insbesondere von 10 bis 14, 13 bis 14, 9 bis 15 und 10 bis 18 C-Atomen. Bevorzugt ist eine Kettenlänge von 10 bis 14 C-Atomen.

Die als Ausgangsverbindungen verwendeten Diphenylalkane sind in üblicher Weise erhältlich durch thermische Chlorierung oder Photochlorierung der entsprechenden Paraffinkohlenwasserstoffe. Die erhaltenen Chlorparaffin-Gemische werden mit Benzol in Gegenwart von $AlCl_3$umgesetzt. Die Dichloralkane reagieren dabei in bekannter Weise zu Diphenylalkane. Dabei werden die Phenylreste in der Kette bevorzugt an den sekundären C-Atomen eingeführt. Aufgrund der unterschiedlichen Siedebereiche lassen sich die Diphenylalkanae von Mono- und Polyphenylalkanen leicht destillativ abtrennen (Fette, Seife, Anstrichmittel, H.-D. Wulf et al., Bd. 69, S. 32 ff [1967]).

Ein zweckmäßiges Ausgangsmaterial sind die bei der technischen Herstellung von linearen Alkyibenzolen anfallenden Diphenylalkane.

Die Diphenylalkane bzw. die technischen Gemische werden unter den angegebenen Bedingungen hydriert:

Die Hyrdrierung kann kontinuierlich oder diskontinuierlich in einstufiger oder zweistufiger Verfahrensweise erfolgen.

Als Hydrierkatalysatoren sind Katalysatoren auf Basis von Edelmetallen, insbesondere des Palladiums oder Platins, auf üblichen Trägern, wie Aluminiumoxid oder Siliciumdioxid, oder auf Basis von Nickel und/oder Nickel-Molybdän zweckmäßig.

Die bevorzugten Katalysatoren sind Raney-Nickel, insbesondere für die einstufige Hydrierung, Nickel-Molybdän auf Magnesiumsilikat und/oder Molybdän-Nickel auf Aluminiumoxid, letztere, wie aus dem Beispiel hervorgeht, für die zweistufige Hydrierung.

Die vollständige Hydrierung kann beispielsweise durch Messung der UV-Absorption festgestellt werden.

Die Dicyclohexylalkane fallen als farb- und geruchloses Öl mit einer gemäß DAB 8 (Deutsches Arzneibuch, 8. Auflage) außerordentlich geringen Extinktion von 0,01 bei 275 nm an. Solch hervorragende Extinktionswerte werden normalerweise an Paraffinölen nicht erreicht. Die Schwefelsäure-Testnote gemäß DAB 8 ist mit <1 hervorragend.

Sie sind als Ölkomponente in pharmazeutischen und kosmetischen Zubereitungen, insbesondere für äußerliche Zwecke aber auch für interne Zubereitungen hervorragend geeignet. Sie sind sehr gut verträglich und zeichnen sich in nicht vorhersehbarer Weise auf der Haut durch eine gute Spreitfähigkeit aus. Dadurch wird die Wasserdampfdurchlässigkeit der Haut nicht gestört und das sogenannte "hot

feeling", das durch die abdeckenden Eigenschaften auf der Haut häufig von Salbengrundlagen verursacht wird, vermieden. Die erfindungsgemäßen Öle sind sehr gut emulgierbar, so daß man bei der praktischen Anwendung häufig mit weniger Emulgator auskommt als üblicherweise verwendet wird. Für zahlreiche öllösliche Inhaltsstoffe, wie Lichtschutzmittel, Vitamine oder ätherische Öle und Parfümen, sind sie hervorragende Lösungsmittel.

Demnach sind auch Gegenstand der vorliegenden Erfindung Zubereitungen für kosmetische oder pharmazeutische Zwecke, die als Ölkomponente Dicylcohexylalkane der Formel I neben üblichen Bestandteilen, Hilfsmitteln und gegebenenfalls Wirkstoffen enthalten, sowie die Verwendung der Dicylcohexylalkane der Formel I in kosmetische und pharmazeutischen Zubereitungen als Ölkomponente.

Zweckmäßige Zubereitungen, insbesondere in Form von Öl-in-Wasser-, und Wasser-in-Öl-Emulsionen oder in Form üblicher Salben, sind beispielsweise die verschiedensten Cremes, wie Hautcremes, Nährcremes, Feuchtigkeitscremes, Babycremes, Sportcremes, Lichtschutzcremes usw.

Es kommen auch Suspensionen, Gele, Lotionen als Zubereitungen in Betracht.

Weiterhin sind beispielsweise zu nennen Zubereitungen für die Haarpflege, wie Haarkuren, Haarsprays usw.

Ebenso lassen sich die erfindungsgemäßen Dicyclohexylalkane in hautpflegenden Ölen, Sonnenschutzölen, Massageölen, Badeölen oder Kinderölen mit gutem Erfolg verwenden.

In den erfindungsgemäßen Zubereitungen können die Dicylcohexylalkane im allgemeinen in Mengen von 0,1 bis 95 Gew.%, bevorzugt 0,1 bis 60 Gew.%, enthalten sein.

Für die meist als Emulsionen, Suspensionen oder Salben vorliegenden Zubereitungen kommen 3 bis 30 Gew.%, bevorzugt 6 bis 20 Gew.% in Betracht. Hautpflegende Öle enthalten zweckmäßigerweise 10 bis 95 Gew.%, bevorzugt 30 bis 60 Gew.%. Geringe Mengen von 0,1 bis 3,0 Gew.% werden bevorzugt aufgrund ihrer weichmachenden und glanzgebenden Eigenschaften in Zubereitungen für die Haarpflege verwendet.

In den verschiedensten Zubereitungen können die erfindungsgemäß zu verwendenden Dicyclohexylalkane zusammen mit den verschiedensten an sich üblichen Träger- und Verdünnungsmitteln, Hilfsmitteln und gegebenenfalls Wirkstoffen problemlos verarbeitet werden.

Zweckmäßige übliche Bestandteile von kosmetischen und pharmazeutischen Zubereitungen sind beispielsweise: anionische, kationische sowie nichtionische Emulgatoren und Emulsionsstabilisatoren, die gleichzeitig Konsistenzgeber oder Gelbildner sein können, wie Polyvinylpyrrolidon, Fettalkohole, Glycerinmonostearat, Polyacrylsäuren, Cellulosederivate und Ethylenoxid-Propylenoxid-Blockpolymere,
feste oder flüssige Ölkomponenten bzw. Fettstoffe mineralischer, pflanzlicher oder tierischer Herkunft, synthetische Esteröle, wie Triglyceridester und Isopropylmyristat,
hydrophile Komponenten, wie Glycerin, Polyethylenglykol und Propylenglykol.

Weiterhin können als Inhaltsstoffe genannt werden beispielsweise Lichtschutzmittel, Bräunungsmittel, Konservierungsmittel, Antioxidantien, Pigmente, Farbstoffe, ätherische Öle und Parfümöle, Vitamine, Pflanzenextrakte, Collagen usw. Diese Stoffe können beispielsweise dem CTFA, Cosmetic Ingredient Dictionary, 3. Auflage, Washington 1982, entnommen werden.

Als spezielle pharmazeutische Zubereitungen sollen beispielsweise Zinksalben, Rheumasalben, Bronchialsalben oder Cremes mit Hydrocortisonacetat, wie sie aus den Beispielen hervorgehen, genannt werden.

Die folgenden Beispiele sollen die Erfindung näher erläutern:

Herstellung von Dicyclohexylalkanen

1. Einstufiges Verfahren

Diphenylalkan der Kettenlänge $C_{10}$bis $C_{14}$ wird in einem Autoklaven in Gegenwart von Raney-Nickel unter einem Wasserstoffdruck von 200 bar bei 280°C 100 Stunden lang hydriert. Der Wasserstoffverbrauch beträgt 689 l $H_2$ (unter Normalbedingungen) pro Liter eingesetztes Diphenylalkan. Die Extinktion des erhaltenen Dicyclohexylalkans bei 260 nm beträgt 0,12 $cm^{-1}$.

2. Zweistufige Hydrierung

a) Diphenylalkan der Kettenlänge $C_{10}$ bis $C_{14}$ wird bei einem Wasser stoffdruck von 95 bar und 350°C in Gegenwart eines Molybdän-Nickel-Katalysators auf Aluminiumoxid kontinuierlich mit einem Durchsatz von 900 g/Stunde hydriert. Die Wasserstoff-Abschleusung beträgt 250 l/Stunde.
Das erhaltene Gemisch weist bei 275 nm eine Extinktion von ca. 200 $cm^{-1}$ auf.

b) Die vollständige Hydrierung erfolgt in Gegenwart eines Nickel-Molybdän-Katalysators auf Magnesiumsilikat bei einem Wasserstoffdruck von 205 bar und 180°C bei einem Durchsatz von 600 g/Stunde und einer Wasserstoff-Abschleusung von 240 l/Stunde.

Die Eigenschaften des erhaltenen Dicyclohexylalkans können der folgenden Tabelle entnommen werden:

| | nach der 1. Stufe | nach der 2. Stufe |
|---|---|---|
| Farbe | farblos | wasserhell |
| Dichte bei 150°C | 0,891 | 0,877 g/cm$^3$ |
| Viskosität bei 40°C | 21,67 | 27,22 mm$^2$/s |
| Viskosität bei 50°C | 14,62 | 17,01 mm$^2$/s*) |
| Viskosität bei 100°C | 3,80 | 4,36 mm$^2$/s |
| Viskositäts-Index | 28 | 37 |
| Flammpunkt COC | 204°C | 204°C |
| $n_D$ 20 | 1,4021 | 1,4789 |
| Stockpunkt | <–50°C | –49°C |
| UV 275 nm | ca. 200 | 0,01 cm$^{-1}$ |
| 295 nm | – | 0,01 cm$^{-1}$ |
| 300 nm | – | 0,01 cm$^{-1}$ |
| $H_2SO_4$-Note | – | <1 |
| Säurezahl | – | 0 |

*) entspricht 2,64° Engler

Anwendungsbeispiele

Rahmenrezepturen mit Dicyclohexylalkanen-$C_{10}$/$C_{14}$. Die angegebenen Teile sind Gewichtsteile.

| Nährcreme (Typ O/W) | |
|---|---|
| $C_{16}$/$C_{18}$-Fettalkohol mit 6 EO (Ethylenoxid) | 2,0 |
| $C_{16}$/$C_{18}$-Fettalkohol mit 25 EO | 2,0 |
| erfindungsgemäße Ölkomponente | 15,0 |
| Cetylalkohol | 2,0 |
| Glycerinmonostearat | 6,0 |
| Erdnußöl | 5,0 |
| Vitaminöl | 1,0 |
| Siliconöl | 0,1 |
| 1,2–Propylenglykol | 3,0 |
| Konservierungsmittel | 0,5 |
| Parfümöl | 0,2 |
| Wasser | 63,2 |

| Feuchtigkeitscreme (Typ O/W) | |
|---|---|
| $C_{16}$/$C_{18}$-Fettalkohol mit 6 EO | 1,5 |
| $C_{16}$/$C_{18}$-Fettalkohol mit 25 EO | 1,5 |
| erfindungsgemäße Ölkomponente | 6,0 |
| Ethylhexansäure-$C_{16}$/$C_{18}$-ester | 6,0 |
| Cetylstearylalkohol | 7,0 |
| 1,2–Propylenglykol | 3,0 |
| Feuchthaltemittel | 2,0 |
| Konservierungsmittel | 0,5 |
| Parfümöl | 0,2 |
| Wasser | 72,3 |

| Babycreme (Typ =/W) | |
|---|---|
| $C_{16}/C_{18}$-Fettalkohol mit 6 EO | 2,0 |
| $C_{16}/C_{18}$-Fettalkohol mit 25 EO | 2,0 |
| Ethylhexansäure-$C_{16}/C_{18}$-ester | 5,0 |
| erfindungsgemäße Ölkomponente | 5,0 |
| Cetylalkohol | 4,0 |
| Glycerinmonostearat | 4,0 |
| (±)-α-Bisabolol | 0,3 |
| Allantoin | 0,2 |
| 1,2–Propylenglykol | 5,0 |
| Konservierungsmittel | 0,5 |
| Parfümöl | 0,2 |
| Wasser | 71,8 |

| Handcreme (Typ O/W) | |
|---|---|
| $C_{16}/C_{18}$-Fettalkohol mit 6 EO | 2,0 |
| $C_{16}/C_{18}$-Fettalkohol mit 25 EO | 2,0 |
| erfindungsgemäße Ölkomponente | 9,0 |
| Cetylalkohol | 5,0 |
| Glycerinmonostearat | 5,0 |
| Siliconöl 350 | 1,0 |
| Polyvinylpyrrolidon vom K-Wert 30 | 1,0 |
| Glycerin | 10,0 |
| Konservierungsmittel | 0,5 |
| Parfümöl | 0,2 |
| Wasser | 64,3 |

| Sportcreme (Typ W/O) | |
|---|---|
| hydriertes Ricinusöl mit 7 EO | 3,0 |
| Ethylhexansäure-$C_{16}/C_{18}$-Ester | 10,0 |
| erfindungsgemäße Ölkomponente | 10,0 |
| mikrokristallines Wachs | 8,0 |
| Lanolinalkohol | 1,0 |
| 1,2–Propylenglykol | 3,0 |
| Konservierungsmittel | 0,5 |
| Parfümöl | 0,3 |
| Wasser | 64,2 |

| Haarkur | |
|---|---|
| $C_{16}/C_{18}$-Fettalkohol mit 6 EO | 1,5 |
| $C_{16}/C_{18}$-Fettalkohol mit 25 EO | 1,5 |
| Cetylstearylalkohol | 3,0 |
| erfindungsgemäße Ölkomponente | 3,0 |
| 1,2–Propylenglykol | 2,0 |
| Coplymer aus Vinylpyrrolidon und Vinylimidazolinmethochlorid | 3,0 |
| Zitronensäure | 0,5 |
| Konservierungsmittel | 0,5 |
| Parfümöl | 0,2 |
| Wasser | 81,8 |

| Haarspray | |
|---|---|
| Filmbildner | 2,5 |
| Ethanol | 20,0 |
| Methylenchlorid | 25,0 |
| erfindungsgemäße Ölkomponente | 0,2 |
| Treibgase | 52,3 |

| Pflegelotion (After Sun Typ O/W) | |
|---|---|
| $C_{16}/C_{18}$-Fettalkohol mit 6 EO | 2,0 |
| $C_{16}/C_{18}$-Fettalkohol mit 25 EO | 2,0 |
| Ethylhexansäure-$C_{16}/C_{18}$-Ester | 7,0 |
| erfindungsgemäße Ölkomponente | 8,0 |
| Cetylalkohol | 1,0 |
| Glycerinmonostearat | 6,0 |
| Siliconöl 100 | 0,1 |
| Panthenol | 1,0 |
| (±)-α-Bisabolol | 0,2 |
| 1,2-Propylenglykol | 3,0 |
| Konservierungsmittel | 0,5 |
| Parfümöl | 0,2 |
| Wasser | 68,9 |

| Sonnenschutzöl | |
|---|---|
| erfindungsgemäße Ölkomponente | 50,0 |
| Ethylhexansäure-$C_{16}/C_{18}$-Ester | 30,0 |
| p-Methoxyzimtsäureethylhexylester | 3,0 |
| $C_{16}/C_{18}$-Fettalkohol mit 25 EO | 2,0 |
| Parfümöl | 0,3 |
| $C_8$–$C_{12}$-Fettsäuretriglycerid | 16,7 |

| Massageöl | |
|---|---|
| erfindungsgemäße Ölkomponente | 60,0 |
| Ethylhexansäure-$C_{16}$/$C_{18}$-Ester | 40,0 |

| Badeöl | |
|---|---|
| erfindungsgemäße Ölkomponente | 52,0 |
| Isopropylmyristat | 43,0 |
| Nonylphenol + 6 EO | 5,0 |

| Körperöle | | |
|---|---|---|
| 1. | erfindungsgemäße Ölkomponente | 60,0 |
| | Ethylhexansäure-$C_{16}$/$C_{18}$-Ester | 20,0 |
| | $C_8$–$C_{12}$-Fettsäuretriglycerid | 20,0 |
| 2. | erfindungsgemäße Ölkomponente | 89,5 |
| | Ethylhexansäure-$C_{16}$/$C_{18}$-Ester | 10,0 |
| | (±)-α-Bisabolol | 0,5 |

| Polyvinylpyrrolidon-Jod-Creme | |
|---|---|
| 10,0 | PVP-Jod, mikronisiert |
| 2,0 | $C_{16}$/$C_{18}$-Fettalkohol mit 6 Mol Ethylenoxid |
| 2,0 | $C_{16}$/$C_{18}$-Fettalkohol mit 25 Mol Ethylenoxid |
| 10,0 | erfindungsgemäße Ölkomponente |
| 10,0 | Cetylstearylalkohol |
| 5,0 | Glycerin |
| 61,0 | Wasser |

| Polyvinylpyrrolidon-Jod-Puderspray | |
|---|---|
| PVP-Jod, mikronisiert | 4,4 |
| erfindungsgemäße Ölkomponente | 1,5 |
| Siliciumdioxid | 1,0 |
| Fluorkohlenwasserstoff-Treibgas ad | 100,0 |

| Zinksalbe | |
|---|---|
| 2,5 | $C_{16}$/$C_{18}$-Fettalkohol mit 25 Mol EO |
| 10,0 | Cetylstearylalkohol |
| 10,0 | Vaseline |
| 10,0 | erfindungsgemäße Ölkomponente |
| 20,0 | Zinkoxid |
| 47,5 | Wasser |

| Rheumasalbe | |
| --- | --- |
| 3,0 | $C_{16}/C_{18}$-Fettalkohol mit 8 Mol EO |
| 3,0 | $C_{16}/C_{18}$-Fettalkohol mit 25 Mol EO |
| 10,0 | Glycerinmonostearat |
| 20,0 | Vaseline |
| 10,0 | erfindungsgemäße Ölkomponente |
| 3,0 | Fichtennadelöl |
| 2,0 | Rosmarinöl |
| 6,0 | Salicylsäuremethylester |
| 1,5 | Nikotinsäurebenzylester |
| 41,5 | Wasser |

| Creme mit Hydrocortisonacetat | |
| --- | --- |
| 2,0 | $C_{16}/C_{18}$-Fettalkohol mit 6 Mol EO |
| 2,0 | $C_{16}/C_{18}$-Fettalkohol mit 25 Mol EO |
| 4,0 | Cetylalkohol |
| 4,0 | Glycerinmonostearat |
| 10,0 | erfindungsgemäße Ölkomponente |
| 5,0 | 1,2-Propylenglykol |
| 1,0 | Hydrocortisonacetat |
| 72,0 | Wasser |

| Bronchialsalbe | |
| --- | --- |
| 10,0 | Stearinsäure mit 9 EO |
| 1,0 | $C_{16}/C_{18}$-Fettalkohol mit 25 Mol EO |
| 5,0 | Glycerinmonostearat |
| 20,0 | Vaseline |
| 10,0 | erfindungsgemäße Ölkomponente |
| 1,0 | Thymianöl |
| 1,0 | Salbeiöl |
| 2,0 | Rosmarinöl |
| 1,0 | Eukalyptusöl |
| 4,0 | Terpentinöl |
| 2,0 | Latschenkiefernöl |
| 3,0 | Menthol |
| 6,0 | Campher |
| 34,0 | Wasser |

| Massagecreme | |
| --- | --- |
| 3,0 | $C_{16}/C_{18}$-Fettalkohol mit 6 Mol EO |
| 1,1 | $C_{16}/C_{18}$-Fettalkohol mit 25 Mol EO |
| 6,0 | Cetylstearylalkohol |
| 30,0 | erfindungsgemäße Ölkomponente |
| 5,0 | Paraffin, weiß, F. 50-55°C |
| 10,0 | 1,2-Propylenglykol |
| 44,9 | Wasser |

## Patentansprüche

1. Dicyclohexylalkane der Formel I

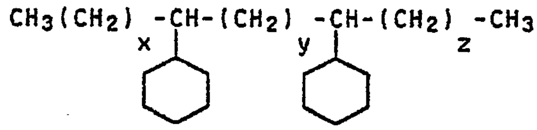

in der x, y und z eine Zahl von 0 bis 24 bedeuten, wobei die Summe x + y + z 4 bis 24 beträgt.

2. Dicyclohexylalkane der Formel I nach Anspruch 1, in der x, y und z eine Zahl von 0 bis 14 bedeuten, wobei die Summe x + y + z 4 bis 14 beträgt.

3. Dicyclohexylalkane der Formel I nach Anspruch 1 oder 2, in denen die Alkylkette insgesamt 10 bis 14 C-Atome enthält.

4. Verfahren zur Herstellung von Dicyclohexylalkanen der Formel I nach Anspruch 1 bis 3, dadurch gekennzeichnet, daß man die zugrundeliegenden Diphenylalkane mit Wasserstoff bei einem Druck von 90 bis 220 bar und bei Temperaturen von 150 bis 350°C in Gegenwart eines Hydrierkatalysators hydriert.

5. Verfahren nach Anspruch 4, dadurch gekennzeichnet, daß man die Hydrierung mit Katalysatoren auf Basis von Nickel oder Nickel-Molybdän durchführt.

6. Zubereitungen für kosmetische oder pharmazeutische Zwecke, die als Ölkomponente Dicyclohexylalkane der Formel I nach Ansprüchen 1 bis 3 enthalten.

7. Zubereitungen nach Anspruch 6, die 0,1 bis 95 Gew.% Dicyclohexylalkane der Formel I enthalten.

8. Verwendung von Dicyclohexylalkanen der Formel I nach Ansprüchen 1 bis 3 als Ölkomponente in kosmetischen oder pharmazeutischen Zubereitungen.

9. Verwendung von Dicyclohexylalkanen der Formel I nach Anspruch 8 in einer Menge von 0,1 bis 95 Gew.% als Ölkomponente in kosmetischen oder pharmazeutischen Zubereitungen.

## Claims

1. A dicyclohexylalkane of the formula I

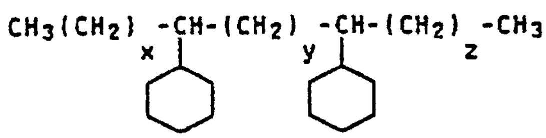

where x, y and z are each from 0 to 24 and the sum x + y + z is from 4 to 24.

2. A dicyclohexylalkane of the formula I as claimed in claim 1, wherein x, y and z are each from 0 to 14 and the sum x + y + z is from 4 to 14.

3. A dicyclohexylalkane of the formula I as claimed in either of claims 1 and 2, wherein the alkyl chain contains a total of from 10 to 14 carbon atoms.

4. A process for the preparation of a dicylohexylalkane of the formula I as claimed in any of claims 1 to 3, wherein the parent diphenylalkane is hydrogenated with hydrogen under from 90 to 220 bar and at from 150 to 350°C in the presence of a hydrogenation catalyst.

5. A process as claimed in claim 4, wherein the hydrogenation is carried out using a catalyst based on nickel or nickel-molybdenum.

6. A formulation for cosmetic or pharmaceutical purposes, which contains, as the oil component, a dicyclohexylalkane of the formula I as claimed in any of claims 1 to 3.

7. A formulation as claimed in claim 6, which contains from 0.1 to 95% by weight of a dicyclohexylalkane of the formula I.

8. Use of a dicyclohexylalkane of the formula I as claimed in any of claims 1 to 3 as the oil component of cosmetic or pharmaceutical formulations.

9. Use of a dicyclohexylalkane of the formula I as claimed in claim 8 in an amount of from 0.1 to 95% by weight as the oil component of cosmetic or pharmaceutical formulations.

## Revendications

1. Dicyclohexylalcanes de formule I

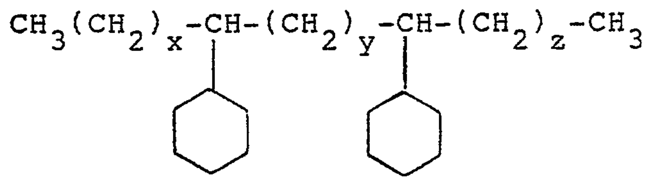

dans laquelle x, y et z représentent un nombre de 0 à 24, la somme x + y + z étant de 4 à 24.

2. Dicyclohexylalcanes de formule I, selon la revendication 1, dans laquelle x, y et z représentent un nombre de 0 à 14, la somme x + y + z étant de 4 à 14.

3. Dicyclohexylalcanes de formule I, selon la revendication 1 ou 2, dans lesquelles la chaîne alkyle contient au total 10 à 14 atomes C.

4. Procédé de préparation de dicyclohexylalcanes de formule I selon les revendications 1 à 3, caractérisé par le fait qu'on hydrogène les diphénylalcanes de base avec de l'hydrogène, à une pression de 90 à 220 bar et des températures de 150° à 350°C en présence d'un catalyseur d'hydrogénation.

5. Procédé selon la revendication 4, caractérisé par le fait qu'on effectue l'hydrogènation avec des catalyseurs à base de nickel ou nickel-molybdène.

6. Préparations à usages cosmétiques ou pharmaceutiques qui contiennent, comme composants huileux, des dicyclohexylalcanes de formule I selon les revendications 1 à 3.

7. Préparations selon la revendication 6, qui contiennent 0,1 à 95% en poids de dicylcohexylalcanes de formule I.

8. Utilisation de dicyclohexylalcanes de formule I selon les revendications 1 à 3, comme composants huileux dans des préparations à usages cosmétiques ou pharmaceutiques.

9. Utilisation de dicyclohexylalcanes de formule I selon la revendication 8 en quantités de 0,1 à 95% en poids, comme composants huileux dans des préparations à usages cosmétiques ou pharmaceutiques.